# EUROPEAN PATENT SPECIFICATION

(11) **EP 4 433 006 B1**
(45) Date of publication and mention of the grant of the patent: **29.04.2026**
(21) Application number: 22802278.6
(22) Date of filing: 31.10.2022
(51) Int. Cl.: A61F 9/008, A61B 3/10

(54) **SCANNING LASER OPHTHALMOSCOPE LASER GUIDANCE FOR LASER VITREOLYSIS**
LASERFÜHRUNG MIT LASER-SCANNING-OPHTHALMOSKOP FÜR DIE LASERVITREOLYSE
GUIDAGE LASER D'OPHTALMOSCOPE LASER À BALAYAGE POUR LA VITRÉOLYSE LASER

(30) Priority: 19.11.2021 US 202163281520 P
(43) Date of publication of application: 25.09.2024
(73) Proprietor: Alcon Inc., 1701 Fribourg (CH)
(72) Inventor: BOR, Zsolt, Lake Forest, California 92630 (US)
(74) Representative: Murgitroyd & Company
(86) International application number: PCT/IB2022/060493
(87) International publication number: WO 2023/089430

(56) References cited:
- WO-A1-2015/171793
- WO-A1-99/58047
- US-A1- 2016 074 221
- US-B2- 6 789 900
- US-B2- 7 703 922

## Description

### TECHNICAL FIELD

The present disclosure relates generally to ophthalmic laser surgical systems, and more particularly to scanning laser ophthalmoscope laser guidance for laser vitreolysis.

### BACKGROUND

In ophthalmic laser surgery, a surgeon may direct a laser beam into the eye to treat the eye. For example, a laser beam may be directed into the vitreous to treat eye floaters. Eye floaters are clumps of collagen proteins that form in the vitreous. These clumps can disturb vision with moving shadows and distortions. The laser beam may be used to fragment the floaters to improve vision.

Reference is made to the documents US2016/074221A1, US7703922B2 and US 6 789 900 B2 which have been cited as exemplary of the background state of the art.

### BRIEF SUMMARY

The scope of the invention is in accordance with the appended claims.

In certain embodiments, an ophthalmic laser surgical system for treating a floater in an eye includes a scanning laser ophthalmoscopy (SLO) device, a treatment laser device, an xy-scanner, and a computer. The SLO device includes a fast photodiode and a confocal filter. The SLO device: directs an SLO beam along an SLO beam path towards a retina of the eye, receives the SLO beam reflected from the eye using the fast photodiode, generates an image from the reflected SLO beam, the image including a floater shadow cast by the floater on the retina, determines an xy-location of the floater shadow according to an xy-scanner, and determines a z-location of the floater relative to the retina using the confocal filter. The treatment laser device directs a laser beam along a laser beam path towards the z-location of the floater. The xy-scanner receives the SLO beam from the SLO device and directs the SLO beam along the SLO beam path towards the xy-location of the floater shadow. The xy-scanner also receives the laser beam from the treatment laser device and directs the laser beam along the SLO beam path towards the xy-location of the floater shadow. The computer controls the SLO device and the treatment laser device.

Embodiments may include none, one, some, or all of the following features:
* The ophthalmic laser surgical system includes an xy-encoder that detects a position of the xy-scanner corresponding to an xy-location expressed in encoder units and reports the xy-location expressed in encoder units as the xy-location of the floater shadow.
* The treatment laser device includes a z-focusing component that receives the z-location of the floater and directs a focal point of the laser beam towards the z-location of the floater along an angular direction of the xy-location of the floater shadow.
* The SLO device scans over a larger angular range of 40 degrees or greater. The SLO device may also scan over a smaller angular range that is smaller than the larger angular range.
* The computer predicts next xy-locations of the floater shadow, including a first xy-location and a second xy-location later than the first xy-location. The computer may instruct the SLO device to direct the SLO beam at the first xy-location of the floater shadow, and to determine the z-location of the floater. The computer may instruct the SLO device to direct the SLO beam at the second xy-location, and may instruct the treatment laser device to direct the laser beam at the z-location of the floater.

In certain embodiments, an ophthalmic laser surgical system for treating a floater in an eye comprises a scanning laser ophthalmoscopy (SLO) device, a treatment device, and a computer. The SLO device comprises a confocal filter with a lens and a pinhole. The SLO device directs an SLO beam along an SLO beam path towards the eye, generates an image of the retina of the eye, where the image shows the floater shadow of the floater cast on the retina, and determines an xy-location of the floater shadow. The SLO device adjusts the position of the focal point of the lens relative to the pinhole of the confocal filter to generate an image of the floater, and determines the z-location of the floater relative to the retina according to the position of the focal point of the lens relative to the pinhole. The treatment laser device directs a laser beam along a laser beam path towards the xy-location and the z-location of the floater. The computer controls the SLO device and the treatment laser device.

Embodiments may include none, one, some, or all of the following features:
* The treatment laser device directs the focal point of the laser beam towards the z-location of the floater along an angular direction of the xy-location of the floater shadow.
* The ophthalmic laser surgical system further comprises an xy-scanner. The xy-scanner receives the SLO beam from the SLO device and directs the SLO beam along the SLO beam path towards the xy-location of the floater shadow. The xy-scanner also receives the laser beam from the treatment laser device and directs the laser beam along the SLO beam path towards the xy-location of the floater shadow. The ophthalmic laser surgical system may include an xy-encoder. The xy-encoder detects the position of the xy-scanner, which corresponds to an encoder xy-location expressed in encoder units, and reports the encoder xy-location as the xy-location of the floater shadow.
* The computer predicts next xy-locations of floater shadow, including a first xy-location and a second xy-location later than the first xy-location. The computer may instruct the SLO device to direct the SLO beam at the first xy-location of the floater shadow and to determine the z-location of the floater. The computer may instruct the SLO device to direct the SLO beam at the second xy-location and may instruct the treatment laser device to direct the laser beam at the z-location of the floater.
* The SLO device scans the SLO beam over a larger angular range of 40 degrees or greater. The SLO device may scan the SLO beam over a smaller angular range that is smaller than the larger angular range.

The invention is defined by the appended claims.

### BRIEF DESCRIPTION OF THE DRAWINGS

FIGURE 1 illustrates an example of an ophthalmic laser surgical system that may be used to treat an eye, according to certain embodiments;
FIGURE 2 illustrates an example of an image that may be generated by the system of FIGURE 1;
FIGURE 3 is an example of scanning laser ophthalmoscope (SLO) device that may be used in the system of FIGURE 1, according to certain embodiments;
FIGURE 4 is a graph illustrating an example of tracking and predicting the xy-location of a floater shadow, which may be used by the system of FIGURE 1, according to certain embodiments; and
FIGURE 5 illustrates an example of a method for treating a floater that may be performed by the system of FIGURE 1, according to certain embodiments.

### DESCRIPTION OF EXAMPLE EMBODIMENTS

Referring now to the description and drawings, example embodiments of the disclosed apparatuses, systems, and methods are shown in detail. The description and drawings are not intended to be exhaustive or otherwise limit the claims to the specific embodiments shown in the drawings and disclosed in the description. Although the drawings represent possible embodiments, the drawings are not necessarily to scale and certain features may be simplified, exaggerated, removed, or partially sectioned to better illustrate the embodiments.

Certain imaging systems may use an optical coherence tomography (OCT) device to image and determine the z-location of floaters in the eye. However, OCT devices are expensive. Accordingly, the systems described herein use a scanning laser ophthalmoscope (SLO) device to measure the z-location of the floater, as well as the xy-location of the floater shadow. The SLO device includes a confocal filter that can be used to determine the z-location of the floater.

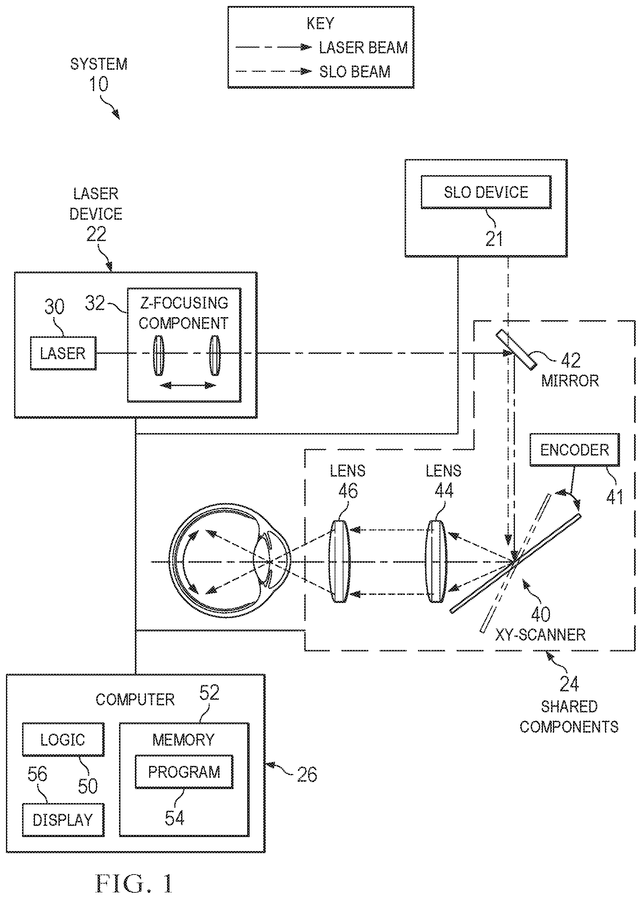

FIGURE 1 illustrates an example of an ophthalmic laser surgical system 10 that may be used to treat an eye, according to certain embodiments. As an overview, system 10 includes a scanning laser ophthalmoscope (SLO) device 21, a treatment laser device 22, one or more shared components 24, and a computer 26, coupled as shown. Treatment laser device 22 includes a laser 30 and a z-focusing component 32, coupled as shown. Shared components 24 include an xy-scanner 40, an xy-encoder 41, and optical elements (such as a mirror 42 and lenses 44 and 46), coupled as shown. Computer 26 includes logic 50, a memory 52 (which stores a computer program 54), and a display 56, coupled as shown.

As an overview of operation of system 10, SLO device 21 directs an SLO beam along an SLO beam path towards a floater shadow and/or floater within an eye and determines an xy-location of the floater shadow according to an xy-scanner and/or a z-location of the floater relative to the retina. Treatment laser device 22 directs a laser beam along a laser beam path towards the z-location of the floater. Shared component xy-scanner 40 receives the SLO beam and the xy-location of the floater shadow and directs the SLO beam along the SLO beam path towards the xy-location of the floater shadow. Xy-scanner 40 also receives the laser beam from treatment laser device 22 and directs the laser beam along the laser beam path aligned with the SLO beam path towards the xy-location of the floater shadow.

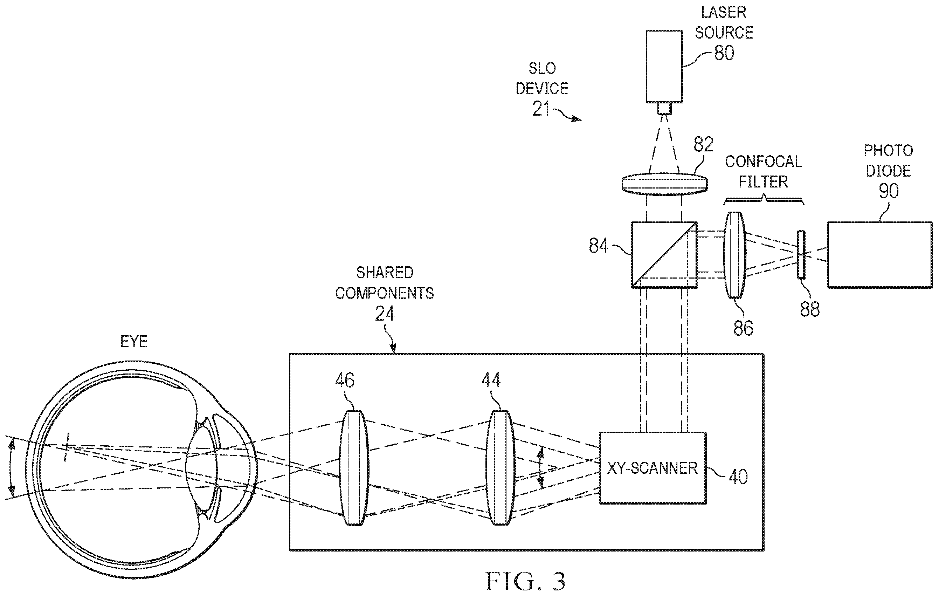

Turning to the parts of the system, SLO device 21 detects, locates, and/or images a floater and/or floater shadow in the vitreous. To image a floater shadow, SLO device 21 directs an SLO beam along an SLO beam path towards the retina, onto which the shadow is cast. SLO device 21 detects the reflected beam and generates an image from the reflected beam, which may be displayed on display 56 as a video. SLO device 21 determines (and may with the help of computer 26) the xy-location of the floater shadow from the image of the shadow. SLO device 21 has a higher image frame rate (e.g., 30 to 60, such 45 to 55, or approximately 50, frames per sec (frames/s)) than that of an interferometer device, which allows for faster tracking of the floater shadow. In addition, SLO device 21 includes a confocal filter that includes a z-focusing component (e.g., a tunable confocal lens) that can be used to measure the z-location of a floater, as described with reference to FIGURE 3.

Turning to treatment laser device 22, ultrashort pulse laser 30 generates a laser beam with any suitable wavelength, e.g., in a range from 400 nm to 2000 nm. Treatment laser device 22 delivers laser pulses at any suitable repetition rate (e.g., a single pulse to 200 megahertz (MHz)). A laser pulse has any suitable pulse duration (e.g., 20 femtoseconds (fs) to 1000 nanoseconds (ns)), any suitable pulse energy (e.g., 1 nanojoule (nJ) to 10 millijoule (mJ)), and a focal point of any suitable size (e.g., 1 to 30 microns (µm)). In a particular embodiment, the laser is a picosecond or femtosecond laser with a repetition rate that exceeds 100 pulses per second (pps).

Z-focusing component 32 longitudinally directs the focal point of the laser beam to a specific location in the direction of the floater shadow. In certain embodiments, z-focusing component 32 receives the z-location of the floater from computer 26 or SLO device 21, and directs the laser beam towards the z-location of the floater. Z-focusing component 32 may include a lens of variable refractive power, a mechanically movable lens, an electrically tunable lens (e.g., Optotune lens), an electrically or mechanically tunable telescope. In certain embodiments, treatment laser device 22 or the optical delivery system also includes a fast xy-scanner used in tandem with z-focusing component 32 to, e.g., create a 3D focal spot pattern. Examples of such scanners include galvo, MEMS, resonant, or acousto-optical scanners.

Shared components 24 direct SLO and laser beams from SLO device 21 and treatment laser device 22, respectively, towards the eye. Because SLO and laser beams both use shared components 24, both beams are affected by the same optical distortions (e.g., fan distortion of scanners, barrel or pillow distortions of the scanner lens, refractive distortions from the inner eye surfaces, and other distortions). The distortions affect both beams in the same way, so the distortions are compensated for. This allows for aiming the laser beam using images generated by the SLO beam with improved accuracy.

As an overview of operation of shared components 24, mirror 42 directs a beam (SLO and/or laser beam) towards xy-scanner 40, which transversely directs the beam towards lens 44. Lenses 44 and 46 direct the beam towards eye. Shared components 24 may also provide spectral and polarization coupling and decoupling of SLO and laser beams to allow the beams to share the same path.

Turning to the details of shared components 24, in certain embodiments, xy-scanner 40 receives the xy-location of the floater shadow from SLO device 20 or computer 26, and directs the SLO and/or laser beam towards the xy-location. Xy-scanner 40 may be any suitable xy-scanner that transversely directs the focal point of the beam in the x- and y-directions and changes the angle of incidence of the beam into the pupil. For example, xy-scanner 40 includes a pair of galvanometrically-actuated scanner mirrors that can be tilted about mutually perpendicular axes. As another example, xy-scanner 40 includes an acousto-optical crystal that can acousto-optically steer the beam. As another example, xy-scanner 40 includes a fast scanner (e.g., a galvo, resonant, or acousto optical scanner) that can create, e.g., a 2D matrix of laser spots.

Xy-encoder 41 detects the position of xy-scanner 40 and reports the position as the xy-location. For example, xy-encoder 41 detects the angular orientations of the galvanometer mirrors of xy-scanner 40 in encoder units. Xy-encoder 41 may report the position in encoder units to SLO device 21, treatment laser device 22, and/or computer 26. Since SLO device 21 and treatment laser device 22 share xy-scanner 40, computer 26 can use the encoder units to instruct system 20 and device 22 where to aim their beams, making it unnecessary to perform the computer-intensive conversion from encoder units to a length unit such as millimeters. Xy-encoder 41 may report the positions at any suitable rate, e.g., once every 5 to 50 milliseconds (ms), such as every 10 to 30 or approximately every 20 ms.

Shared components 24 also include optical elements. In general, an optical element can act on (e.g., transmit, reflect, refract, diffract, collimate, condition, shape, focus, modulate, and/or otherwise act on) a laser beam. Examples of optical elements include a lens, prism, mirror, diffractive optical element (DOE), holographic optical element (HOE), and spatial light modulator (SLM). In the example, optical elements include mirror 42 and lenses 44 and 46. Mirror 42 may be a trichroic mirror. Lenses 44 and 46 may be scanning optics of an SLO device.

Computer 26 controls components of system 10 (e.g., SLO device 21, treatment laser device 22, and/or shared components 24) in accordance with a computer program 54. Computer 26 may be separated from components or may be distributed among system 10 in any suitable manner, e.g., within SLO device 21, treatment laser device 22, and/or shared components 24. In certain embodiments, portions of computer 26 that control SLO device 21, treatment laser device 22, and/or shared components 24 may be part of SLO device 21, treatment laser device 22, and/or shared components 24, respectively.

Computer 26 controls and may be part of the components of system 10 to allow the components to perform their operations. Computer 26 controls the components in accordance with a computer program 54. Examples of computer programs 54 include floater shadow imaging, floater shadow tracking, image processing, floater evaluation, retinal exposure calculation, patient education, and insurance authorization programs. For example, computer 26 may use a computer program 54 to instruct and/or control SLO device 21, treatment laser device 22, and/or shared components 24 to image a floater shadow or a floater, determine the xy- and z-locations of the floater, and focus a laser beam at the floater.

In certain embodiments, computer 26 uses an image processing program 54 to perform image processing on an image, e.g., analyze the digital information of the image to extract information from the image. In certain embodiments, image processing program 54 analyzes an image of a floater shadow to obtain information about the floater. For example, program 54 may detect a floater shadow by detecting a darker shape in an image (using, e.g., edge detection or pixel analysis). As another example, program 54 may detect the shape and size of a floater shadow, which indicate the size and shape of the floater. As another example, program 54 may detect the tone or luminescence of the floater shadow, which indicates the density of the floater.

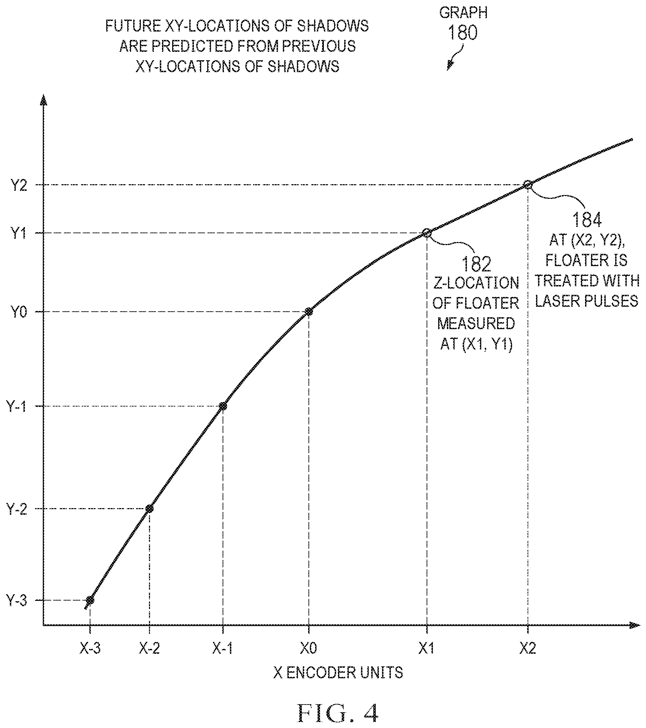

In certain embodiments, computer 26 uses a tracking program 54 to track and/or predict the movement of a floater shadow. For example, computer 26 may perform image analysis of retinal video from the SLO device to track the movement of the floater shadow. Tracking program 54 may predict the movement of the floater shadow and send to treatment laser device 22 the location of where the floater shadow is predicted to be when the laser beam reaches the floater. In certain embodiments, computer 26 may perform tracking and/or predicting operations as a part of SLO device 21. Tracking and predicting floater shadow movement is described in more detail with reference to FIGURE 4.

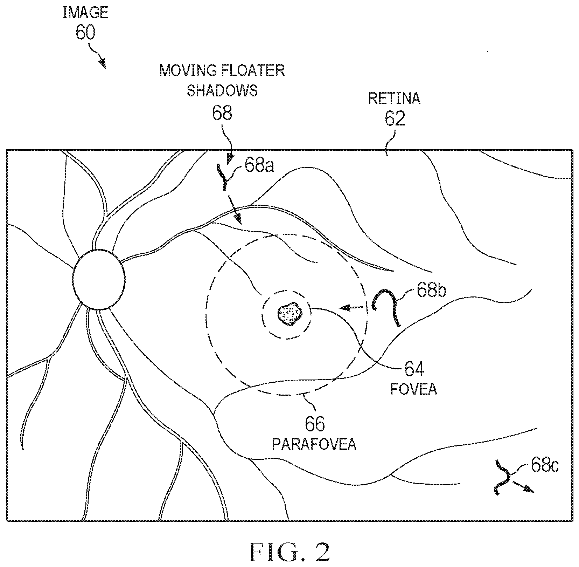

FIGURE 2 illustrates an example of a retinal image 60 that may be generated by system 10 of FIGURE 1. Image 60 shows the retina 62 of an eye, with a foveal region (or fovea) 64 and a parafoveal region (or parafovea) 66. Generally, fovea 64 has a visual angle of approximately +/- one degree, and parafovea 66 has a visual angle of approximately +/- seven degrees. Image 60 also shows examples of floater shadows 68 (68a, 68b, 68c) that floaters cast on retina 62. Other floater shadows may have a different size, shape, and/or optical density.

A floater may move when the gaze of the patient moves. Most floaters are embedded in the vitreous, a viscous substance, so the movement is relatively slow and predictable, with no unexpected accelerating or sudden stopping. Accordingly, tracking floater shadows is technically feasible. Non-moving shadows are generally not caused by floaters, and may be caused by, e.g., corneal or lens opacities or anatomical changes of the retina, so floater treatment is not concerned with non-moving shadows.

A floater may be regarded as clinically significant if it can cause a visual disturbance, which can be determined from any suitable features of the floater shadow, e.g., the size and/or density of the shadow, proximity of the shadow to the fovea and/or parafovea, and/or the track of the shadow relative to the fovea and/or parafovea. As an example, a floater can cause a visual disturbance if it permanently or transiently casts a shadow 68 on fovea 64 or can cause distraction or annoyance if it permanently or transiently casts a shadow 68 on parafovea 66. Accordingly, if a floater shadow falls within or is predicted to move within fovea 64 and/or parafovea 66, the floater may be designated as clinically significant. As another example, floater shadow 68 can be used to estimate the size and density of the floater. Larger, denser floaters are more likely to cause a visual disturbance. Thus, a shadow 68 larger than a critical shadow size can indicate a clinically significant floater. A shadow 68 with a higher contrast relative to the background may indicate a clinically significant floater.

In certain embodiments, a user such as a surgeon may determine significance from the displayed images (such as a video) of the floater shadow. An image processing program can assist the user in making the decision. In other embodiments, the computer can use image processing and target evaluation computer programs to determine significance from the image.

FIGURE 3 is an example of scanning laser ophthalmoscope (SLO) device 21 that may be used in system 10 of FIGURE 1, according to certain embodiments. SLO device 21 includes a tunable confocal lens that can measure the z-location of a floater. In the example, SLO device 21 includes a laser source 80, a collimating lens 82, a beam splitter 84, a confocal filter, (comprising a z-scanner lens 86 and a pinhole 88), and a photodiode 90, coupled as shown.

As an example of operation, laser source 80 generates an SLO beam, and collimating lens 82 collimates the beam. Beam splitter 84 transmits the beam from laser source 80 to shared components 24. Xy-scanner 40 (which includes a pair of scanner mirrors) deflects the beam towards the eye. Xy-scanner 40 may scan the SLO beam over any suitable angular range, e.g., a larger range such as at least 30, 40, 50, or 120 degrees, or a smaller range less than the larger range. Lenses 44 and 46 conjugate the scanner mirrors into the pupil of the eye. The SLO beam enters the eye through the pupil. The natural lens of the eye focuses the SLO beam onto the retina of the eye.

The natural lens of the eye and lenses 44 and 46 collimate the back-reflected light from the retina. Xy-scanner 40 and beamsplitter 84 direct the back-reflected light towards the confocal filter, which includes lens 86 and pinhole 88. Depending on the position of the focal point of lens 86 relative to pinhole 88, different parts of the interior of the eye can be imaged. For example, if the focal point of lens 86 is located at pinhole 88, the confocal filter transmits only the focused SLO beam reflected from the retina (which can show floater shadows) and suppresses the other light. As another example, the focal point of lens 86 may be placed before pinhole 88 in order to visualize a floater and suppress the other back-reflected light. Photodiode 90 measures the intensity of the back-reflected light.

Computer 20 records the angular orientation of the mirrors of xy-scanner 40 and the intensity of the back-reflected light as measured by photodiode 90. Computer 20 uses this information to generate an image of the retina or floater. The speed of a computer 26 of a standard SLO is typically high enough to display the retinal image as a real-time video.

Turning to the components, laser source 80 may be any suitable laser that generates an SLO beam, which may comprise white light. The confocal filter directs light towards photodiode 90 and includes lens 86 and pinhole 88. Lens 86 may be any suitable scanner or lens that can change the focus of light in the z-direction, e.g., an electrically tunable lens. The distance between the focal point of lens 86 and pinhole 88 ("ZCF distance") can be adjusted to image different parts of the eye interior in any suitable manner. For example, the ZCF distance may be mechanically adjusted. As another example, lens 86 may be an electrically tunable lens (e.g., an Optotune lens) that can adjust its focal point.

Photodiode 90 may be any suitable light detector, e.g., an avalanche photodiode (APD). Photodiode 90 may be a fast photodiode, such as a photodiode with a response time in the range of 0.1 nanosecond (ns) to 1 microsecond (us), such as 1 ns to 1 us. The response time may be selected according to desired sensitivity. In certain embodiments, photodiode 90 may allow for a video frame rate of up to 25 frame /sec with a spatial resolution of approximately 20 micrometers (um)).

In certain embodiments, computer 20 and/or SLO device 21 can utilize information from the confocal filter to determine the distance between the floater and the retina ("FR distance") in order to focus the treatment laser onto a floater. In the embodiments, computer 20 accesses optical software to convert the ZCF distance to the FR distance. Computer 20 uses the FR distance to adjust z-focusing component 32 of laser device 22 to focus the treatment laser beam onto the floater.

FIGURE 4 is a graph 180 illustrating an example of tracking and predicting the xy-location of a floater shadow, which may be used by ophthalmic laser surgical system of FIGURE 1, according to certain embodiments. In the embodiments, a computer uses a tracking program to track and/or predict the movement of a floater shadow. For example, the computer performs image analysis of retinal images to track the movement of the floater shadow to track the floater. As discussed with reference to FIGURE 2, floater treatment is concerned with moving shadows.

In the example, the xy-location is given in encoder units (which may be provided by encoder 41). Variable t represents time t = -3, -2, -1, 0, 1, 2, where t = 0 is the current time, t = -3, -2, -1 is the past time, and t = 1, 2, 3 is the future time. Yt represents the y-location in encoder units at time t, and Xt represents the x-location in encoder units at time. The tracking program may predict the future xy-locations from extrapolations of past xy-locations.

In the example, the confocal filter, which includes lens 86 and pinhole 88, measures the z-location of the floater at the xy-location (x1, y1) at time t = 1, shown at reference number 182. The measurement may be performed a few milliseconds prior to firing the laser. At the xy-location (x2, y2) at time t = 2, shown at reference number 184, the treatment laser device fires a laser beam comprising laser pulses. The laser beam is directed by the xy-scanner to the xy-location (x2, y2) and is focused by the z-focusing component at the z-location. In general, floaters do not move much in the z-direction, so the z-location measured at t = 1 may be sufficiently close to the z-location at t = 2.

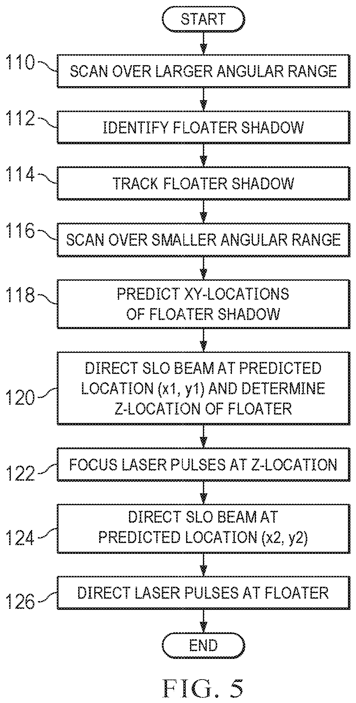

FIGURE 5 illustrates an example of a method for treating a floater that may be performed by system 10 of FIGURE 1, according to certain embodiments. The method starts at step 110, where the SLO device scans over a larger angular range to generate images of the retina. For example, the SLO may scan an angular range of 40 degrees or greater with a frame rate of 50 frame per second (frame/s) or greater.

The computer identifies the xy-location of the floater shadow from the images at step 112. The SLO device tracks the floater shadow at step 114. The SLO device scans over a smaller angular range at step 116. For example, the SLO may scan a smaller angular range of 5 to 20 degrees. The smaller angular range allows for a faster frame rate.

The computer predicts the xy-locations of the floater shadow at step 118 at future SLO frames during which the z-location of the floater can be measured and laser pulses can be directed to the floater. For example, the computer may predict the xy-locations at the next 2 to 5 frames, e.g., the xy-locations (x1, y1) and (x2, y2) at the next frames 1 and 2 may be predicted. In certain embodiments, an encoder provides the location of the shadow in encoder units, and the computer may predict future shadow locations from past shadow locations.

The SLO device directs the SLO beam at predicted location (x1, y1) and the confocal filter measures the z-location of the floater at step 120. The treatment laser device focuses laser pulses at the z-location of the floater at step 122. For example, the computer instructs the z-focusing component of the laser device to focus pulses at the z-location. The SLO device directs the SLO beam at the predicted location (x2, y2) at step 124. During the duration of steps 120 and 124, floaters typically move in the xy-direction, with negligible movement in the z-direction.

The treatment laser device directs laser pulses at the floater at step 126 to fragment the floater. Floaters have a volume with dimensions of typically a few millimeters. In certain embodiments, the laser pulses may form a two-dimensional (2D) or three-dimensional (3D) pattern of any suitable number of pulses, which may range from a few tens to a few thousand pulses, and may depend in the size of the floater. Any suitable repetition rate may be used. For example, a repetition rate of several kHz may yield a treatment time that is less than one second.

A component (such as the control computer) of the systems and apparatuses disclosed herein may include an interface, logic, and/or memory, any of which may include computer hardware and/or software. An interface can receive input to the component and/or send output from the component, and is typically used to exchange information between, e.g., software, hardware, peripheral devices, users, and combinations of these. A user interface is a type of interface that a user can utilize to communicate with (e.g., send input to and/or receive output from) a computer. Examples of user interfaces include a display, Graphical User Interface (GUI), touchscreen, keyboard, mouse, gesture sensor, microphone, and speakers.

Logic can perform operations of the component. Logic may include one or more electronic devices that process data, e.g., execute instructions to generate output from input. Examples of such an electronic device include a computer, processor, microprocessor (e.g., a Central Processing Unit (CPU)), and computer chip. Logic may include computer software that encodes instructions capable of being executed by an electronic device to perform operations. Examples of computer software include a computer program, application, and operating system.

A memory can store information and may comprise tangible, computer-readable, and/or computer-executable storage medium. Examples of memory include computer memory (e.g., Random Access Memory (RAM) or Read Only Memory (ROM)), mass storage media (e.g., a hard disk), removable storage media (e.g., a Compact Disk (CD) or Digital Video or Versatile Disk (DVD)), database, network storage (e.g., a server), and/or other computer-readable media. Particular embodiments may be directed to memory encoded with computer software.

Although this disclosure has been described in terms of certain embodiments, modifications (such as changes, substitutions, additions, omissions, and/or other modifications) of the embodiments will be apparent to those skilled in the art. Accordingly, modifications may be made to the embodiments without departing from the scope of the invention. For example, modifications may be made to the systems and apparatuses disclosed herein. The components of the systems and apparatuses may be integrated or separated, or the operations of the systems and apparatuses may be performed by more, fewer, or other components, as apparent to those skilled in the art. As another example, modifications may be made to the methods disclosed herein. The methods may include more, fewer, or other steps, and the steps may be performed in any suitable order, as apparent to those skilled in the art.

To aid the Patent Office and readers in interpreting the claims, Applicants note that they do not intend any of the claims or claim elements to invoke 35 U.S.C. §112(f), unless the words "means for" or "step for" are explicitly used in the particular claim. Use of any other term (e.g., "mechanism," "module," "device," "unit," "component," "element," "member," "apparatus," "machine," "system," "processor," or "controller") within a claim is understood by the applicants to refer to structures known to those skilled in the relevant art and is not intended to invoke 35 U.S.C. §112(f).

## Claims

1. An ophthalmic laser surgical system (10) for treating a floater in an eye, comprising:
a scanning laser ophthalmoscopy, SLO, device (21) comprising a fast photodiode (90) and a confocal filter, the SLO device (21) configured to:
direct an SLO beam along an SLO beam path towards a retina of the eye;
receive the SLO beam reflected from the eye using the fast photodiode (90);
generate an image from the reflected SLO beam, the image including a floater shadow cast by the floater on the retina;
determine an xy-location of the floater shadow according to an xy-scanner; and
determine a z-location of the floater relative to the retina using the confocal filter; and
a treatment laser device (22) configured to direct a laser beam along a laser beam path towards the z-location of the floater;
the xy-scanner (40) configured to:
receive the SLO beam from the SLO device (21) and direct the SLO beam along the SLO beam path towards the xy-location of the floater shadow; and
receive the laser beam from the treatment laser device and direct the laser beam along the SLO beam path towards the xy-location of the floater shadow; and
a computer (20) configured to control the SLO device and the treatment laser device.

2. The ophthalmic laser surgical system of Claim 1, further comprising an xy-encoder (41) configured to:
detect a position of the xy-scanner (40), the position corresponding to an encoder xy-location expressed in encoder units; and
report the encoder xy-location expressed in encoder units as the xy-location of the floater shadow.

3. The ophthalmic laser surgical system of Claim 1, the treatment laser device (22) comprising a z-focusing component (32) configured to:
receive the z-location of the floater; and
direct a focal point of the laser beam towards the z-location of the floater along an angular direction of the xy-location of the floater shadow.

4. The ophthalmic laser surgical system of Claim 1, the SLO device (21) configured to:
scan over a larger angular range of 40 degrees or greater.

5. The ophthalmic laser surgical system of Claim 4, the SLO device (21) configured to:
scan over a smaller angular range that is smaller than the larger angular range.

6. The ophthalmic laser surgical system of Claim 1, the computer (20) configured to:
predict a plurality of next xy-locations of floater shadow, the plurality of next xy-locations of floater shadow comprising a first xy-location and a second xy-location later than the first xy-location.

7. The ophthalmic laser surgical system of Claim 6, the computer (20) configured to instruct the SLO device (21) to:
direct the SLO beam at the first xy-location of the floater shadow; and
determine the z-location of the floater.

8. The ophthalmic laser surgical system of Claim 6, the computer (20) configured to:
instruct the SLO device (21) to direct the SLO beam at the second xy-location; and
instruct the treatment laser device (22) to direct the laser beam at the z-location of the floater.

9. An ophthalmic laser surgical system for treating a floater in an eye, comprising:
a scanning laser ophthalmoscopy, SLO, device (21) comprising a confocal filter, the confocal filter comprising a lens (86) and a pinhole (88), the SLO device (21) configured to:
direct an SLO beam along an SLO beam path towards the eye;
generate an image of a retina of the eye, the image showing a floater shadow of the floater cast on the retina;
determine an xy-location of the floater shadow;
adjust a position of a focal point of the lens relative to the pinhole of the confocal filter to generate an image of the floater; and
determine a z-location of the floater relative to the retina according to the position of the focal point of the lens (86) relative to the pinhole (88);
a treatment laser device (22) configured to direct a laser beam along a laser beam path towards the xy-location and the z-location of the floater; and
a computer configured to control the SLO device (21) and the treatment laser device (22).

10. The ophthalmic laser surgical system of Claim 9, the treatment laser device (22) configured to:
direct a focal point of the laser beam towards the z-location of the floater along an angular direction of the xy-location of the floater shadow.

11. The ophthalmic laser surgical system of Claim 9, further comprising an xy-scanner (40) configured to:
receive the SLO beam from the SLO device and direct the SLO beam along the SLO beam path towards the xy-location of the floater shadow; and
receive the laser beam from the treatment laser device and direct the laser beam along the SLO beam path towards the xy-location of the floater shadow.

12. The ophthalmic laser surgical system of Claim 11, further comprising an xy-encoder (41) configured to:
detect a position of the xy-scanner (40), the position corresponding to an encoder xy-location expressed in encoder units; and
report the encoder xy-location expressed in encoder units as the xy-location of the floater shadow.

13. The ophthalmic laser surgical system of Claim 9, the computer (20) configured to:
predict a plurality of next xy-locations of floater shadow, the plurality of next xy-locations of floater shadow comprising a first xy-location and a second xy-location later than the first xy-location.

14. The ophthalmic laser surgical system of Claim 13, the computer (20) configured to instruct the SLO device (21) to:
direct the SLO beam at the first xy-location of the floater shadow; and
determine the z-location of the floater.

15. The ophthalmic laser surgical system of Claim 13, the computer (20) configured to:
instruct the SLO device (21) to direct the SLO beam at the second xy-location; and
instruct the treatment laser device (22) to direct the laser beam at the z-location of the floater.

## Patentansprüche

1. Ophthalmisches Laserchirurgiesystem (10) zur Behandlung eines Floaters in einem Auge, das Folgendes umfasst:
Laser-Scanning-Ophthalmoskopie (SLO)-Vorrichtung (21), die eine schnelle Photodiode (90) und ein Konfokalfilter umfasst, wobei die SLO-Vorrichtung (21) für Folgendes ausgebildet ist:
Richten eines SLO-Strahls entlang eines SLO-Strahlpfads zu einer Netzhaut des Auges;
Empfangen des SLO-Strahls, der von dem Auge reflektiert wird, unter Verwendung der schnellen Photodiode (90);
Erzeugen eines Bilds aus dem reflektierten SLO-Strahl, wobei das Bild einen Floater-Schatten beinhaltet, der durch den Floater auf die Netzhaut geworfen wird;
Bestimmen einer xy-Position des Floater-Schattens gemäß einem xy-Scanner; und
Bestimmen einer z-Position des Floaters relativ zur Netzhaut unter Verwendung des Konfokalfilters; und
eine Behandlungslaservorrichtung (22), die dazu ausgebildet ist, einen Laserstrahl entlang eines Laserstrahlpfads in Richtung der z-Position des Floaters zu richten;
wobei der xy-Scanner (40) zu Folgendem ausgebildet ist: Empfangen des SLO-Strahls von der SLO-Vorrichtung (21) und Richten des SLO-Strahls entlang des SLO-Strahlpfads auf die xy-Position des Floater-Schattens; und
Empfangen des Laserstrahls von der Behandlungslaservorrichtung und Richten des Laserstrahls entlang des SLO-Strahlpfads auf die xy-Position des Floater-Schattens; und
einen Computer (20), der dazu ausgebildet ist, die SLO-Vorrichtung und die Behandlungslaservorrichtung zu steuern.

2. Ophthalmisches Laserchirurgiesystem nach Anspruch 1, das ferner einen xy-Codierer (41) umfasst, der für Folgendes ausgebildet ist:
Detektieren einer Position des xy-Scanners (40), wobei die Position einer Codierer-xy-Position entspricht, die in Codierereinheiten ausgedrückt ist; und
Melden der xy-Position des Codierers, die in Codierereinheiten ausgedrückt wird, als die xy-Position des Floater-Schattens.

3. Ophthalmisches Laserchirurgiesystem nach Anspruch 1, wobei die Behandlungslaservorrichtung (22) eine z-fokussierende Komponente (32) umfasst, die zu Folgendem ausgebildet ist:
Bestimmen einer xy-Position des Floaters; und
Richten eines Brennpunkts des Laserstrahls entlang einer Winkelrichtung der xy-Position des Floater-Schattens auf die z-Position des Floaters.

4. Ophthalmisches Laserchirurgiesystem nach Anspruch 1, wobei die SLO-Vorrichtung (21) für Folgendes ausgebildet ist:
Scannen über einen größeren Winkelbereich von 40 Grad oder mehr.

5. Ophthalmisches Laserchirurgiesystem nach Anspruch 4, wobei die SLO-Vorrichtung (21) für Folgendes ausgebildet ist:
Scannen über einen kleineren Winkelbereich, der kleiner als der größere Winkelbereich ist.

6. Ophthalmisches Laserchirurgiesystem nach Anspruch 1, wobei der Computer (20) für Folgendes ausgebildet ist:
Vorhersagen einer Vielzahl von nächsten xy-Positionen des Floater-Schattens, wobei die Vielzahl der nächsten xy-Positionen des Floater-Schattens eine erste xy-Position und eine zweite xy-Position später als die erste xy-Position umfassen.

7. Ophthalmisches Laserchirurgiesystem nach Anspruch 6, wobei der Computer (20) dazu ausgebildet ist, die SLO-Vorrichtung (21) zu Folgendem anzuweisen:
Richten des SLO-Strahls auf die erste xy-Position des Floater-Schattens; und
Bestimmen der z-Position des Floaters.

8. Ophthalmisches Laserchirurgiesystem nach Anspruch 6, wobei der Computer (20) für Folgendes ausgebildet ist:
Anweisen der SLO-Vorrichtung (21), den SLO-Strahl auf die zweite xy-Position zu richten; und
Anweisen der Behandlungslaservorrichtung (22), den Laserstrahl auf die z-Position des Floaters zu richten.

9. Ophthalmisches Laserchirurgiesystem zum Behandeln eines Floaters in einem Auge, das Folgendes umfasst:
Laser-Scanning-Ophthalmoskopie (SLO)-Vorrichtung (21), die ein Konfokalfilter umfasst, wobei das Konfokalfilter eine Linse (86) und ein Pinhole (88) umfasst, wobei die SLO-Vorrichtung (21) für Folgendes ausgebildet ist:
Richten eines SLO-Strahls entlang eines SLO-Strahlpfads zum Auge;
Erzeugen eines Bilds einer Netzhaut des Auges, wobei das Bild einen Floater-Schatten des Floaters zeigt, der auf die Netzhaut geworfen wird;
Bestimmen einer xy-Position des Floaters; und
Anpassen einer Position eines Brennpunkts der Linse relativ zum Pinhole des Konfokalfilters, um ein Bild des Floaters zu erzeugen; und
Bestimmen einer z-Position des Floaters relativ zu der Netzhaut gemäß der Position des Brennpunkts der Linse (86) relativ zu dem Pinhole (88);
eine Behandlungslaservorrichtung (22), die dazu ausgebildet ist, einen Laserstrahl entlang eines Laserstrahlpfads in Richtung der xy-Position und der z-Position des Floaters zu richten; und
einen Computer, der dazu ausgebildet ist, die SLO-Vorrichtung (21) und die Behandlungslaservorrichtung (22) zu steuern.

10. Ophthalmisches Laserchirurgiesystem nach Anspruch 9, wobei die Behandlungslaservorrichtung (22) für Folgendes ausgebildet ist:
Richten eines Brennpunkts des Laserstrahls entlang einer Winkelrichtung der xy-Position des Floater-Schattens auf die z-Position des Floaters.

11. Ophthalmisches Laserchirurgiesystem nach Anspruch 9, das ferner einen xy-Scanner (40) umfasst, der für Folgendes ausgebildet ist:
Empfangen des SLO-Strahls von der SLO-Vorrichtung und Richten des SLO-Strahls entlang des SLO-Strahlpfads auf die xy-Position des Floater-Schattens; und
Empfangen des Laserstrahls von der Behandlungslaservorrichtung und Richten des Laserstrahls entlang des SLO-Strahlpfads auf die xy-Position des Floater-Schattens.

12. Ophthalmisches Laserchirurgiesystem nach Anspruch 11, das ferner einen xy-Codierer (41) umfasst, der für Folgendes ausgebildet ist:
Detektieren einer Position des xy-Scanners (40), wobei die Position einer Codierer-xy-Position entspricht, die in Codierereinheiten ausgedrückt ist; und
Melden der xy-Position des Codierers, die in Codierereinheiten ausgedrückt wird, als die xy-Position des Floater-Schattens.

13. Ophthalmisches Laserchirurgiesystem nach Anspruch 9, wobei der Computer (20) für Folgendes ausgebildet ist:
Vorhersagen einer Vielzahl von nächsten xy-Positionen des Floater-Schattens, wobei die Vielzahl der nächsten xy-Positionen des Floater-Schattens eine erste xy-Position und eine zweite xy-Position später als die erste xy-Position umfassen.

14. Ophthalmisches Laserchirurgiesystem nach Anspruch 13, wobei der Computer (20) dazu ausgebildet ist, die SLO-Vorrichtung (21) zu Folgendem anzuweisen:
Richten des SLO-Strahls auf die erste xy-Position des Floater-Schattens; und
Bestimmen der z-Position des Floaters.

15. Ophthalmisches Laserchirurgiesystem nach Anspruch 13, wobei der Computer (20) für Folgendes ausgebildet ist:
Anweisen der SLO-Vorrichtung (21), den SLO-Strahl auf die zweite xy-Position zu richten; und
Anweisen der Behandlungslaservorrichtung (22), den Laserstrahl auf die z-Position des Floaters zu richten.

## Revendications

1. Système chirurgical ophtalmique à laser (10) destiné au traitement d'un corps flottant dans un œil, comprenant :
un dispositif d'ophtalmoscopie laser à balayage, SLO, (21) comprenant une photodiode rapide (90) et un filtre confocal, le dispositif SLO (21) étant configuré pour :
diriger un faisceau SLO le long d'un trajet de faisceau SLO vers une rétine de l'œil ;
recevoir le faisceau SLO réfléchi en provenance de l'œil en utilisant la photodiode rapide (90) ;
générer une image à partir du faisceau SLO réfléchi, l'image comportant une ombre de corps flottant projetée par le corps flottant sur la rétine ;
déterminer un emplacement xy de l'ombre de corps flottant en fonction d'un scanner xy ; et
déterminer une position z du corps flottant par rapport à la rétine en utilisant le filtre confocal ; et
un dispositif laser de traitement (22) configuré pour diriger un faisceau laser le long d'un trajet de faisceau laser vers l'emplacement z du corps flottant ;
le scanner xy (40) étant configuré pour :
recevoir le faisceau SLO en provenance du dispositif SLO (21) et diriger le faisceau SLO le long du trajet du faisceau SLO vers l'emplacement xy de l'ombre de corps flottant ; et
recevoir le faisceau laser en provenance du dispositif laser de traitement et diriger le faisceau laser le long du trajet du faisceau SLO vers l'emplacement xy de l'ombre de corps flottant ; et
un ordinateur (20) configuré pour commander le dispositif SLO et le dispositif laser de traitement.

2. Système chirurgical ophtalmique à laser selon la revendication 1, comprenant en outre un encodeur xy (41) configuré pour :
détecter une position du scanner xy (40), la position correspondant à un emplacement xy d'encodeur exprimé en unités d'encodeur ; et
indiquer l'emplacement xy d'encodeur exprimé en unités d'encodeur comme emplacement xy de l'ombre de corps flottant.

3. Système chirurgical ophtalmique à laser selon la revendication 1, le dispositif laser de traitement (22) comprenant un composant de focalisation en z (32) configuré pour :
recevoir l'emplacement z du corps flottant ; et
diriger un point focal du faisceau laser vers l'emplacement z du corps flottant le long d'une direction angulaire de l'emplacement xy de l'ombre de corps flottant.

4. Système chirurgical ophtalmique à laser selon la revendication 1, le dispositif SLO (21) étant configuré pour :
balayer une plage angulaire supérieure de 40 degrés ou plus.

5. Système chirurgical ophtalmique à laser selon la revendication 4, le dispositif SLO (21) étant configuré pour :
balayer une plage angulaire inférieure qui est inférieure à la plage angulaire supérieure.

6. Système chirurgical ophtalmique à laser selon la revendication 1, l'ordinateur (20) étant configuré pour :
prédire une pluralité d'emplacements xy suivants d'ombre de corps flottant, la pluralité d'emplacements xy suivants d'ombre de corps flottant comprenant un premier emplacement xy et un deuxième emplacement xy postérieur au premier emplacement xy.

7. Système chirurgical ophtalmique à laser selon la revendication 6, l'ordinateur (20) étant configuré pour commander au dispositif SLO (21) de :
diriger le faisceau SLO au premier emplacement xy de l'ombre de corps flottant ; et
déterminer l'emplacement z du corps flottant.

8. Système chirurgical ophtalmique à laser selon la revendication 6, l'ordinateur (20) étant configuré pour :
commander au dispositif SLO (21) de diriger le faisceau SLO au deuxième emplacement xy ; et
commander au dispositif laser de traitement (22) de diriger le faisceau laser à l'emplacement z du corps flottant.

9. Système chirurgical ophtalmique à laser destiné au traitement d'un corps flottant dans un œil, comprenant :
un dispositif d'ophtalmoscopie laser à balayage, SLO, (21) comprenant un filtre confocal, le filtre confocal comprenant une lentille (86) et un sténopé (88), le dispositif SLO (21) étant configuré pour :
diriger un faisceau SLO le long d'un trajet de faisceau SLO vers l'œil ;
générer une image d'une rétine de l'œil, l'image montrant une ombre de corps flottant du corps flottant projetée sur la rétine ;
déterminer un emplacement xy du corps flottant ; et
ajuster une position d'un point focal de la lentille par rapport au sténopé du filtre confocal pour générer une image du corps flottant ; et
déterminer un emplacement z du corps flottant par rapport à la rétine en fonction de la position du foyer de la lentille (86) par rapport au sténopé (88) ;
un dispositif laser de traitement (22) configuré pour diriger un faisceau laser le long d'un trajet de faisceau laser vers l'emplacement xy et l'emplacement z du corps flottant ; et
un ordinateur configuré pour commander le dispositif SLO (21) et le dispositif laser de traitement (22).

10. Système chirurgical ophtalmique à laser selon la revendication 9, le dispositif laser de traitement (22) étant configuré pour :
diriger un point focal du faisceau laser vers l'emplacement z du corps flottant le long d'une direction angulaire de l'emplacement xy de l'ombre de corps flottant.

11. Système chirurgical ophtalmique à laser selon la revendication 9, comprenant en outre un scanner xy (40) configuré pour :
recevoir le faisceau SLO en provenance du dispositif SLO et diriger le faisceau SLO le long du trajet du faisceau SLO vers l'emplacement xy de l'ombre de corps flottant ; et
recevoir le faisceau laser en provenance du dispositif laser de traitement et diriger le faisceau laser le long du trajet du faisceau SLO vers l'emplacement xy de l'ombre de corps flottant.

12. Système chirurgical ophtalmique à laser selon la revendication 11, comprenant en outre un encodeur xy (41) configuré pour :
détecter une position du scanner xy (40), la position correspondant à un emplacement xy d'encodeur exprimé en unités d'encodeur ; et
indiquer l'emplacement xy d'encodeur exprimé en unités d'encodeur comme emplacement xy de l'ombre de corps flottant.

13. Système chirurgical ophtalmique à laser selon la revendication 9, l'ordinateur (20) étant configuré pour :
prédire une pluralité d'emplacements xy suivants d'ombre de corps flottant, la pluralité d'emplacements xy suivants d'ombre de corps flottant comprenant un premier emplacement xy et un deuxième emplacement xy postérieur au premier emplacement xy.

14. Système chirurgical ophtalmique à laser selon la revendication 13, l'ordinateur (20) étant configuré pour commander au dispositif SLO (21) de :
diriger le faisceau SLO au premier emplacement xy de l'ombre de corps flottant ; et
déterminer l'emplacement z du corps flottant.

15. Système chirurgical ophtalmique à laser selon la revendication 13, l'ordinateur (20) étant configuré pour :
commander au dispositif SLO (21) de diriger le faisceau SLO au deuxième emplacement xy ; et
commander au dispositif laser de traitement (22) de diriger le faisceau laser à l'emplacement z du corps flottant.
